# EUROPEAN PATENT APPLICATION

(11) **EP 4 488 381 A1**
(43) Date of publication of application: **08.01.2025**
(21) Application number: 23184072.9
(22) Date of filing: 07.07.2023
(51) Int. Cl.: C12Q 1/6806

(54) **MODIFIED DNA IDENTIFIER SEQUENCES FOR DNA MODIFICATION SCREENING**

(71) Applicant: ETH Zurich, 8092 Zurich (CH)
(72) Inventor: STURLA, Shana, 8053 Zürich (CH); PÜLLEN, Nikolai, 8057 Zürich (CH); TAKHAVEEV, Vakil, 5000 Aarau (CH)

(57) **Abstract**

The present invention is directed to modified DNA identifier sequences (MoDIS) comprising (i) a validation code nucleotide sequence (VC), (ii) a randomized index code nucleotide sequence (RIC) and (iii) an annealing site nucleotide sequence (AS) for specifically binding a primer for PCR amplification, wherein (a) AS is attached to an affinity tag or marker and/or affinity tag compound for the identification, enrichment and/or purification of MoDIS-bound nucleotide sequences; and (b) the nucleotide at the 5'-end of the MoDIS comprises a 5'-azido-modified moiety or a 5'-alkynyl-modified moiety.

## Description

The present invention is directed to modified DNA identifier sequences (MoDIS) comprising (i) a validation code nucleotide sequence (VC), (ii) a randomized index code nucleotide sequence (RIC) and (iii) an annealing site nucleotide sequence (AS) for specifically binding a primer for PCR amplification, wherein (a) AS is attached to a marker and/or affinity tag compound for the identification, enrichment and/or purification of MoDIS-bound nucleotide sequences; and (b) the nucleotide at the 5'-end of the MoDIS comprises a 5'-azido-modified moiety or a 5'-alkynyl-modified moiety.

The invention further pertains to a method for detecting chemical modifications in DNA sequences comprising the steps of extraction of DNA from a biological sample; enzymatic treatment of the extracted DNA with at least one protein for nucleotide excision to remove chemically modified nucleotides and to yield an at least one-nucleotide-long gap in the double-stranded DNA with a free 3'-hydroxyl moiety; introduction of 3'-(*O*-propargyl)-modified nucleotides (prop-dNTP) or 3'-azido-modified nucleotides at the free 3'-hydroxyl sites of the extracted DNA to provide 3'-propargyl-modified DNA or 3'-azido-modified-DNA, respectively; either reaction of the 3'-(*O-*propargyl)-modified nucleotides of the extracted DNA with 5'-azido-modified nucleotides of the MoDIS to form triazole-linked DNA nucleotide sequences comprising the MoDIS linked via triazole to the extracted DNA fragments; or alternatively, reaction of the 3'-azido-modified nucleotides of the extracted DNA with 5'-alkynyl-modified nucleotides of MoDIS to form triazole-linked DNA nucleotide sequences comprising the MoDIS linked via triazole to the extracted DNA fragments; enrichment and/or purification of the triazole-linked DNA; amplification of the triazole-linked DNA by polymerase chain reaction (PCR) by means of a primer binding to the annealing site (AS) of the MoDIS; and sequencing of the amplified triazole-linked DNA nucleotides. The invention further encompasses uses of the MoDIS, kits of parts comprising these as well as methods for the identification of DNA damage and/or the diagnosis of a disease or medical condition in a mammal.

Nucleobase modifications in DNA serve a valuable purpose in the form of epigenetic marks which are formed through enzymatic machineries and control gene expression. However, modifications can also be harmful by causing mutations or genome instability and are commonly termed DNA damage or lesion. Over 70,000 lesions are generated daily per cell in the form of more than hundred different nucleobase modifications caused by replication errors, chemical assault, or radiation. *(Lindahl et al. 2000, (Chatterjee et al. 2017)* DNA lesions are ultimately involved in the progress of ageing and chronic diseases like cancer.

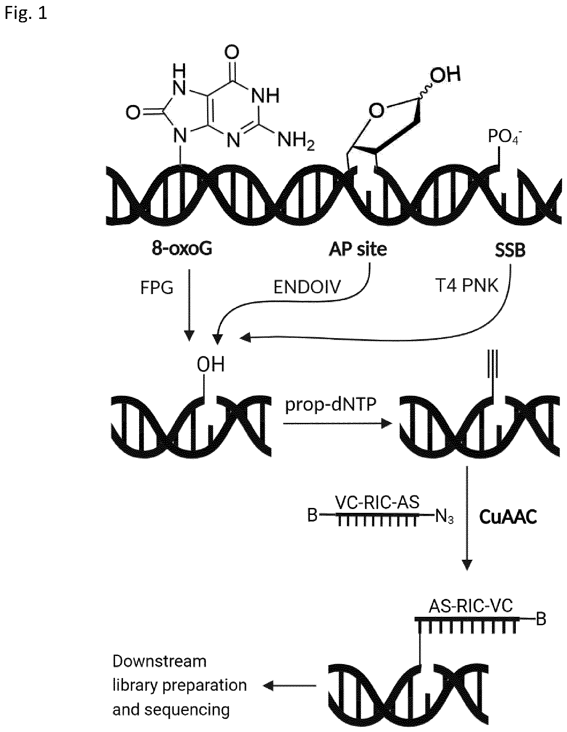

Accurate damage sequencing is challenging because the variety and similarity of DNA modifications aggravate specific targeting. Some of the most prevalent lesions in genomic DNA are single strand breaks (SSBs), apurinic sites (AP sites) and oxidative modifications as 8-oxoguanine (8-oxoG) (see Fig. 1). *(Swenberg et al. 2011), (Tubbs et al. 2017)*

Methods for DNA modification (damage) sequencing include antibody-based approaches, direct chemical tagging and pulldown-based methods, enzymatic damage excision, enzymatic ligation and polymerase stalling at the modification site. *(Mingard et al. 2020)*

More recently developed methods to map damage sites include GLOE-Seq and SSiNGLe for SSBs, AP-Seq and nick-Seq for AP sites and click-code-seq or DPC-seq for oxidative lesions *(Poetsch et al. 2018), (Wu et al. 2018), (Cao et al. 2019, (Cao et al. 2020, (Sriramachandran et al. 2020), (Tang et al. 2022).*

All these methods rely on the presence or generation of a SSB with a 3'-OH end at the damage site for sequencing adapter ligation. This 3'-OH can be produced through chemical tagging, polymerase stalling or enzymatic excision of the damaged nucleobase. However, naturally abundant SSBs and termini in DNA can be confused with the 3'-OH of the damage site generating false positive reads. Commonly, oligonucleotide sequences for downstream sequencing (adapters) are enzymatically ligated to the 3'-OH of the damage site, which can be inefficient and/or require the use of additional oligonucleotides (splinters). Depending on the design of the adapter and damage detection strategy, methods are typically limited to the mapping of a few or even just one DNA modification. In routine PCR for final library amplification, false positive reads can be generated by unspecific binding of primers that should bind the adapter but in fact also bind segments of the genome. Since this is unavoidable due to the high complexity of the genome, strategies for filtering these false positive reads are required, but are not implemented in most published sequencing methods. Another pitfall of applying PCR is the loss of original quantitative information, i.e. the actual number of damage sites, since true damage reads cannot be distinguished from PCR duplicates. Finally, applying antibodies to enrich true positive damage reads, makes damage mapping protocol significantly more cumbersome, time- and cost expensive and is limited to the availability, quality and specificity of the antibody.

Wu *et al.* developed a method for generating single-nucleotide-resolution maps of oxidatively damaged guanine in the yeast genome employing click chemistry for efficient adapter ligation. *(Wu et al. 2018)* The damage mapping is only qualitative, providing binary-type information as to whether a certain guanine is oxidatively modified or not. The method does not allow for determining the frequency of chemical modifications in genomes of a population of cells. Specifically, this method is applied to yeast genomic DNA, in which chemically modified deoxyguanosine-phosphates are excised, resulting in a free 3'-OH, to which a DNA polymerase attaches a 3'-(O-propargyl)-dGTP. Further in the method, this 3'-alkynyl-modified genomic sequence is reacted with 5'-azido-modified code sequence (L-P5-azido) to form a triazole which is compatible with downstream PCR amplification. To multiply the DNA comprising the triazole-bound genomic and code sequences by PCR and adapt it for sequencing, a PCR primer (P5-uni) is used that binds to the code sequence and covers the triazole link. One of the major disadvantages in this design of the code sequence and the primer is the artefact formation during PCR because the primer also frequently binds to genomic nucleotide sequences, thus resulting in frequent false positive calling of DNA modification sites.

In view of the above discrepancies in the art, it is the objective underlying the present invention to provide tools and methods for identifying, localizing, and quantifying chemical modifications in DNA, optionally in genomic DNA of preferably mammalian and human origin, with high resolution and low false positives.
The above objectives are solved by modified DNA identifier sequences (MoDIS) having the structure
(a) 5'-VC - RIC - AS-3',
(b) 5'-RIC - VC - AS-3',
(c) 5'-VCp1 - RIC - VCp2 - AS-3',
(d) 5'-RICp1 - VC - RICp2 - AS-3', or
(e) 5'-VCp1 - RICp1 - VCp2 - RICp2 - AS-3'
wherein
(i) VC is a validation code nucleotide sequence having 3 or more fixed nucleotides located upstream of RIC (a), downstream of RIC (b), or located in part upstream (VCp1) and located in part downstream of RIC (VCp2) (c);
(ii) RIC is a randomized index code nucleotide sequence having 3 or more randomly selected nucleotides (a-c), wherein the RIC can be located in part upstream (RICp1) and in part downstream (RICp2) of VC (d, e);
(iii) AS is an annealing site nucleotide sequence for specifically binding a primer for PCR amplification,
wherein
(a) the annealing site nucleotide sequence (AS) is attached to a marker and/or affinity tag compound for the identification, enrichment and/or purification of MoDIS-bound nucleotide sequences; and
(b) the nucleotide at the 5'-end of the MoDIS comprises a 5'-azido-modified moiety or a 5'-alkynyl-modified moiety.

The term "modified DNA identifier sequences (MoDIS)", as used herein, is meant to refer to functionalized DNA nucleotide sequences, specifically to a group, combination or composition of/comprising or consisting of DNA nucleotide sequences that are identical in sequence except for the varying randomized index code nucleotide sequence (RIC) elements. For example, the group can comprise at least one, two or more different RIC elements. Optionally, the modified DNA identifier sequences (MoDIS) may vary in their VC and AS elements to the extent that the function of these is not compromised.

It is further noted that the sequence of the modified DNA identifier sequences (MoDIS) may comprise further nucleotides that may vary in length and nucleotide code to the extent that the functions of VC, RIC and AS is not impaired.

The modified DNA identifier sequences (MoDIS) of the present invention have the advantage that they can be applied in methods of the invention to identify, localize, and quantify chemical modifications in DNA sequences, even in very large genomic (6×10⁹ bases) and mitochondrial DNA sequences, with high resolution and less false positives compared to the presently available methods and tools. The MoDIS of the present and related methods are particularly suited for determining the location and frequency of chemical modifications in genomic sequences, for genome-wide mapping (i.e., identification and localization) and frequency estimation of oxidative, hydrolysis and alkylation modifications (also referred to as damage) in a DNA, optionally in human and other mammalian genomes. Genome mapping and frequency determination of oxidative, hydrolysis and alkylation damage in DNA are important indicators (optionally, affinity tag or marker) for health status, aging progression, cancer risk, cancer diagnosis, toxicological screenings, and for testing interventions for diseases and aging.

The term "modified DNA", as used herein in the context of the term MoDIS, is meant to refer to any chemical modification of DNA, which modifications encompass but are not limited to all deviations from the four canonical nucleobases (guanine, cytosine, adenine, thymine) in DNA, including products of their oxidation, reduction, alkylation and hydrolysis as well as apurinic/- apyrimidinic sites, also known as AP site or abasic sites, and DNA breaks. For example, and representative are DNA modifications including 8-oxoguanine (8-oxoG), AP, DNA breaks, 2,6-diamino-4-hydroxy-5-formamidopyrimidine (FapyG), 1,*N*6-ethenoadenine (εA), 4,6-diamino-5-formamidopyrimidine (FapyA), 5-formyluracil, 5-hydroxyuracil, 5-hydroxycytosine, thymine glycol, 3-methyladenine, 3-methylguanine, 7-methylguanine, 6-O-methylguanine, 2-O-methylguanine, 2-0-methylcytosine, 0-2-methylthymine, 7-(2-hydroxyethyl)guanine (7-HEG), uracil, hypoxanthine, 5-hydroxymethyluracil. The MoDIS of the present invention are specifically useful for identifying and quantifying chemical DNA modifications relating to 8-oxoguanine (8-oxoG), AP, DNA breaks, 2,6-diamino-4-hydroxy-5-formamidopyrimidine (FapyG), and 1,*N*6-ethenoadenine (εA).

The term "identifier sequences" forming part of the term MoDIS is meant to refer to DNA sequences suitable for DNA polymerization and identification and encompasses all DNA modifications suitable for subsequent DNA polymerization and identification in a method of the present invention.

The term "randomized index code" (RIC), as used herein, is meant to indicate a randomly selected nucleotide sequence which is suitable for identifying and quantifying unique DNA modification occurrences in PCR-amplified genome sequences labeled by MoDIS. Using a mixture of multiple MoDIS different in their RIC allows for identifying and allocating PCR-multiplied genomic sequences to different cells and genomes in a sample. Thus, RIC is essential for estimating the frequency of a DNA modification at certain genomic positions, making the method employing MoDIS quantitative.

The term "validation code" (VC), as used herein, is meant to describe a nucleotide sequence which does not vary in multiple DNA modification identifier sequences (MoDIS) and which marks and validates an MoDIS-bound DNA fragment of interest, i.e. genomic DNA previously characterized by a chemical modification. When a primer for PCR amplification correctly binds and initiates polymerization of the complementary strand from the annealing site sequence (AS) in the MoDIS, the validation code (VC) will indicate that the polymerized nucleotide sequence started at the correct position. VC will thus allow distinguishing any artefacts that would arise when the complementary strand polymerization started in the wrong position along a sequence of interest. In the method employing MoDIS, sequencing readouts that contain VC are validated as identifying a chemical modification and those readouts that do not contain VC are false positives.

The annealing site nucleotide sequence (AS) allows for a PCR-amplification primer to bind specifically, initiating the synthesis of the complementary strand that covers VC confirming the correct position of the primer binding. Optionally, the AS sequence may contain additional nucleotides not involved in primer binding. The AS sequence is attached to an affinity tag compound for the identification, enrichment and/or purification of a MoDIS-bound nucleotide sequence. The affinity tag is positioned at the 3'-end of the AS sequence or within the AS sequence, not hindering the PCR amplification.

The alkynyl moiety of a 3'-alkynyl (e.g. O-propargyl)-modified nucleotide will react with the azido moiety of a 5'-azido-modified nucleotide to form a triazole linker between both nucleotides. A triazole linker within a nucleotide sequence has the advantage that it will essentially not hinder polymerase activity. Both reactive groups are suitable for chemically binding the MoDIS and a DNA fragment of interest.

If a chemically modified nucleotide is enzymatically removed in a double strand nucleotide sequence, the gap can be filled with a 3'-alkynyl (e.g. O-propargyl)-modified nucleotide or, alternatively, with a 3'-azido-modified nucleotide.

When the 5'-end of the MoDIS comprises a 5'-azido-modified nucleotide, it will react with a DNA fragment, wherein the chemically modified nucleotide has been excised and a 3'-alkynyl (e.g. *O*-propargyl)-modified nucleotide has been added to result in a triazole connecting the DNA fragment of interest and the MoDIS at the site of previous chemical damage.

If the 5'-end of the MoDIS comprises a 5'alkynyl-modified nucleotide, it will react with a DNA fragment, wherein the chemically modified nucleotide has been excised and a 3'-azido-modified nucleotide has been added to result in a triazole connecting the DNA fragment of interest and the MoDIS at the site of previous chemical damage.

The RIC, the VC, and the AS of the MoDIS can vary substantially in length as long as these elements remain functional. For example, the randomized index code (RIC) may be a nucleotide sequence having 5 to 200, 5 to 30, 6 to 15 or about 10 random DNA nucleotides. For example, the validation code (VC) may be a nucleotide sequence having 4 to 400, 4 to 100, 4 to 20, 5 to 10 or about 7 random DNA nucleotides. For example, the annealing site (AS) sequence may be a nucleotide sequence having 5 to 50, 10 to 50, 10 to 30, 18 to 30 or about 23 DNA nucleotides.

For practicing the invention, the annealing site nucleotide sequence AS will specifically bind a primer for DNA polymerase-mediated PCR amplification to be used in a method of the present invention, for example and not limited to an AS binding a primer for DNA polymerase-mediated PCR amplification selected from the group consisting of Vent (exo-) DNA polymerase, Deep Vent DNA Polymerase, Q5 high-fidelity DNA polymerase (New England Biolabs, USA), Therminator Polymerase, Therminator IX Polymerase, Klenow fragment, phi29 DNA Polymerase, Sulfolobus DNA Polymerase IV, Taq DNA Polymerase, BST DNA Polymerase (Full length or large fragment), Phusion DNA Polymerase, T7 DNA Polymerase, DNA Polymerase I, T4 DNA Polymerase. *(Chien et al. 1976), (Engler et al. 1983), (Blanco et al. 1984), (Kunkel et al. 1987), (D'Alessio et al. 1988), (Derbyshire et al. 1988), (Jannasch et al. 1992), (Chester et al. 1993), (Kong et al. 1993), (Aliotta et al. 1996), (Gardner et al. 1999), (Notomi et al. 2000), (Boudsocq et al. 2001), (Gardner et al. 2019, (Biolabs 2023).*

For practicing the invention, the affinity tag encompassed by the MoDIS for the identification, enrichment and/or purification of a MoDIS-bound nucleotide sequence may be, for example, selected from the group consisting of biotin, biotin-TEG (triethylene glycol), biotin-Cx, wherein C_{X} is an aliphatic linker, e.g. a C₃, C₁₂ or C₁₈ aliphatic linker), biotin-BB, wherein BB is tertbutyl-benzoyl, to reduce undesired side reactions during oligonucleotide synthesis, or biotin-PC, wherein PC is any photocleavable linker. *(Olejnik et al. 1995, (Nam et al. 2002, (Deng et al. 2003, (Fang et al. 2003, (Cozzolino et al. 2021)* Moreover, immuno-tags can be added to the DNA, e.g. digoxigenin, which can then be captured during a respective antibody in an affinity-based purification method. *(Kessler 1991)*

In a further aspect the present invention is directed to a method for detecting chemical modifications in DNA sequences comprising the steps
(a) Extraction of DNA from a biological sample;
(b) Enzymatic treatment of the extracted DNA with at least one protein for nucleotide excision, optionally selected from the group of proteins consisting of glycosylases, endonucleases, phosphatases, to remove chemically modified nucleotides and to yield an at least one-nucleotide-long gap in the double-stranded DNA with a free 3'-hydroxyl moiety;
(c) Introduction of 3'-(*O*- or S-alkynyl)-, optionally propargyl-modified nucleotides or 3'-azido-modified nucleotides at the free 3'-hydroxyl sites of the extracted DNA to provide 3'-(*O*- or *S*-alkynyl)-modified DNA, optionally 3'-propargyl-modified DNA, or 3'-azido-modified-DNA, respectively;
(d) Optionally, DNA fragmentation with sonication or enzymatically;
(e) Optionally, ligation of adapters suitable for the sequencing technology used, *optionally* containing primer binding sites for loop-circle amplification for sequencing-by-synthesis approaches;
(d1) Reaction of the 3'-(*O*- or S-alkynyl)-, optionally propargyl-modified nucleotides of the extracted DNA with 5'-azido-modified nucleotides of the DNA modification identifier sequences (MoDIS) according to any one of claims 1 to 6 to form triazole-linked DNA nucleotide sequences comprising the DNA modification identifier sequences (MoDIS) linked via triazole to the extracted DNA fragments; or
(d2) Reaction of the 3'-azido-modified nucleotides of the extracted DNA with 5'-(*O*- orS-alkynyl)-, optionally 5'-propargyl-modified nucleotides of DNA modification identifier sequences (MoDIS) according to any one of claims 1 to 6 to form triazole-linked DNA nucleotide sequences comprising the DNA modification identifier sequences (MoDIS) linked via triazole to the extracted DNA fragments;
(e) Enrichment and/or purification of the triazole-linked DNA;
(f) Amplification of the triazole-linked DNA by polymerase chain reaction (PCR) by means of a primer binding to the Annealing site (AS) of the DNA modification identifier sequences (MoDIS); and
(g1) Optionally, indexing with additional commercially supplied adapters for the chosen sequencing technology
(g2) Sequencing of the amplified triazole-linked DNA nucleotides.

The method of the present invention can be used to identify, localize, and quantify chemical modifications in DNA, optionally in genomic or mitochondrial DNA of preferably mammalian and human origin, with high resolution and less false positives than prior art methods.

Optionally, the 3'-(O- orS-alkynyl)-, are C₂₋₁₀ alkynyl, optionally ethynyl, propargyl, pentynyl or octenyl-modified nucleotides.

The extraction of DNA from a biological sample in step (a) can be achieved by conventional methods well known in the field, for example by phenol-chloroform extraction, chemical lysis (e.g. using guanidinium thiocyanate) in combination with silica-based column purification, magnetic bead-based purification, anion exchange chromatography, desalting or cesium chloride density gradients. *(Garger et al. 1983, (Alberti et al. 1990, (Budelier et al. 2001, (Berensmeier 2006, (Sambrook et al. 2006, (Dairawan 2020)*

Biological samples for DNA extraction optionally comprise cells, tissue, feces, blood, and/or saliva. The extracted DNA may optionally be genomic DNA or mitochondrial DNA.

After extraction step (a), for example, background DNA modifications can optionally be blocked to prevent their undesired mapping. For example, if oxidative DNA modifications are to be mapped AP sites and DNA breaks can be blocked, optionally by treating the extracted DNA with apurinic/apyrimidinic (AP) endonuclease 1 (optionally, Endonuclease IV, Endonuclease III) *(Ljungquist 1980), (Rogers et al. 1980), (AI-Attar et al. 2010)* and adding ddNTPs to free 3'-OH groups with a DNA polymerase. *(Gardner et al. 2019)*

In step (b) of the method of the invention the extracted DNA is enzymatically treated with at least one protein for nucleotide excision to remove chemically modified nucleotides and to yield an at least one-nucleotide-long gap in the double-stranded DNA with a free 3'-hydroxyl moiety. The excision enzyme is optionally selected from the group consisting of glycosylases, endonucleases, phosphatases. For example, at least one nucleotide excision protein is selected from the group consisting of formamidopyrimidine DNA glycosylase (Fpg), human apurinic/apyrimidinic endonuclease (APE1), 8-oxo-guanine glycosylase 1 (OGG1), 8-oxo-guanine glycosylase 2 (OGG2), Endonuclease three homologue 1 (NTH1), Endonuclease VII-like 1 / Nei-like DNA glycosylase 1 (NEIL1), Endonuclease VII-like 2 / Nei-like DNA glycosylase 2 (NEIL2), Endonuclease VII-like 3 / Nei-like DNA glycosylase 3 (NEIL3), archaeal GO glycosylase (AGOG), Alkyladenine glycosylase Methylpurine glycosylase (AAG/MPG), 3-meA glycosylase 1 (Tagl), DNA-3-methyladenine glycosylase 2 (AlkA), AlkC, AlkD, Uracil DNA N-glycosylase (UNG), Thymine DNA glycosylase (TDG), ss-selective monofunctional uracil DNA glycosylase 1 (SMUG1), and methyl-CpG-binding domain 4 (MBD4) and T4 polynucleotide kinase (T4 PNK), antarctic phosphatase, alkaline phosphatase, shrimp alkaline phosphatase and pyrophosphatase, as well as mutants of the aforementioned enzymes with improved efficiency, specificity and selectivity. *(Kaplan 1972, (Cameron et al. 1977, (Ljungquist 1980, (Rogers et al. 1980, (Chetsongo et al. 1981, (Hatahet et al. 1994, (Nwaka et al. 1995, (van der Kemp et al. 1996, (Dizdaroglu et al. 1999, (Bollen et al. 2000, (Rina et al. 2000, (Boorstein et al. 2001, (Free et al. 2001, (de Backer et al. 2002, (Sartori et al. 2004, (Alseth et al. 2006, (Al-Attar et al. 2010, (Faucher et al. 2010, (Leitner-Dagan et al. 2012, (Ebrohimkhani et al. 2014, (Yang et al. 2017, (Kisiala et al. 2018, (Kladova et al. 2019, (Sarker et al. 2021, (Servius et al. 2023).*

In step (c) of the method of the invention 3'-(*O*- or *S*-alkynyl)-, optionally propargyl-modified nucleotides (alkyne-/propargyl-dNTP) or 3'-azido-modified nucleotides are introduced at the free 3'-hydroxyl sites of the extracted DNA to provide 3'-alkynyl-, optionally 3'-propargyl-modified DNA, or 3'-azido-modified-DNA, respectively. The alkynyl moiety of the 3'-alkynyl-modified DNA must be capable of reacting with an azido moiety to form a triazole-linked DNA. For example, further O-alkynyl-options for the modified nucleotides include but are not limited to ethynyl, pentynyl, or octenyl-moieties like dibenzocyclooctin. *(Winter et al. 2009, (El-Sagheer et al. 2010, (Uszczynska et al. 2012, (Haque et al. 2014, (Kim et al. 2019)*

The 3-'(*O*- or S-alkynyl)-, optionally propargyl-modified nucleotides (alkyne-/prop-dNTP) or 3'-azido-modified nucleotides can be introduced at the free 3'-hydroxyl sites of the extracted DNA by a DNA polymerase as Therminator DNA Polymerase or similar poylmerases. *(Chien et al. 1976, (Derbyshire et al. 1988, (Aliotta et al. 1996, (Gardner et al. 2019, (Biolabs 2023)*

There are two alternatives for practicing step (d).
(d1) The 3'-(*O*- or *S*-alkynyl)-, optionally propargyl-modified nucleotides of the extracted DNA are reacted with 5'-azido-modified nucleotides of the DNA modification identifier sequences (MoDIS) of the present invention to form triazole-linked DNA nucleotide sequences comprising the DNA modification identifier sequences (MoDIS) linked via triazole to the extracted DNA fragments; or
(d2) The 3'-azido-modified nucleotides of the extracted DNA are reacted with the 5'-alkynyl-, optionally 5'-*O*-propargyl-, modified nucleotides of DNA modification identifier sequences (MoDIS) of the present invention to form triazole-linked DNA nucleotide sequences comprising the DNA modification identifier sequences (MoDIS) linked via triazole to the extracted DNA fragments.

These alkynyl azide-based triazole-linking reactions are known to those of average skill in the art of organic and DNA chemistry. *(El-Sagheer et al. 2010, (Haque etal. 2014, (Kim etal. 2019)*

In the next step (e) the triazole-linked DNA is enriched and/or further purified, for example, by means of an affinity tag compound attached to the annealing site nucleotide sequence (AS). Conventional methods in the art of DNA technology, in particular those based on affinity tags can be applied, for example biotin, biotin-TEG, wherein TEG is triethylene glycol, biotin-C_{X}, wherein C_{X} is a C₃₋₂₀ aliphatic linker, e.g. a C₃, C₁₂ or C₁₈ linker, biotin-BB, wherein BB tertbutyl-benzoyl, e.g. for reducing undesired side reactions during oligonucleotide synthesis, or biotin-PC, wherein PC is a photocleavable linker. *(Olejnik et al. 1995, (Nam et al. 2002, (Deng et al. 2003, (Fang et al. 2003, (Cozzolino et al. 2021)* Moreover, immuno-tags can be added to the DNA, e.g. digoxigenin, which can then be captured during a respective antibody in an affinity-based purification method.

### (Kessler 1991)

Optionally, and before alternative steps (d1) or (d2) the 3'-alkynyl-, optionally 3'-propargyl-modified DNA or 3'-azido-modified-DNA may be fragmented, for example, enzymatically or sonification. *(Elsner et al. 1989, (Sapojnikova et al. 2017)* To use the currently most common DNA sequencing approach, sequence by synthesis, DNA needs to be fragmented to an average fragment size of around 500 bp. Sequencing technologies based on single-molecule real-time sequencing do not require a fragmentation step

Optionally, ligation of adapters suitable for the sequencing technology used.

After enrichment and/or purification the triazole-linked DNA is amplified in step (f) by polymerase chain reaction (PCR) by means of a primer binding to the Annealing site (AS) of the DNA modification identifier sequences (MoDIS) using conventional PCR methods. *(Hoeijmokers et al. 2011, (Shao et al. 2011, (Quail et al. 2012, (Rahman et al. 2013, (EI-Sagheer et al. 2015)* Then the amplified triazole-linked DNA nucleotides are optionally ligated to sequence technology-specific indexing adapters. The amplified triazole-linked DNA sequenced and analyzed to identify, and quantify the nature, position, and frequency of DNA modifications in the extracted DNA.

Optionally, the extracted DNA for practicing the method of the present invention is genomic DNA or mitochondrial DNA, and, for example, extracted cells, feces, tissue, blood, or saliva

In specific and optional embodiments, the method of the present invention is one, wherein
(i) the 3'-(*O*- or *S*-alkynyl)-, optionally propargyl-modified nucleotides of the extracted DNA are reacted with 5'-azido-modified nucleotides of the DNA modification identifier sequences (MoDIS) of the present invention, or
(ii) the 3'-azido-modified nucleotides of the extracted DNA are reacted with the 5'-(*O*- or *S-*alkynyl)-, optionally propargyl-modified nucleotides of the DNA modification identifier sequences (MoDIS) of the present invention,
to form triazole-linked DNA nucleotide sequences.

For example, the method of the invention is optionally one, wherein enrichment and/or purification of the triazole-linked DNA nucleotides is based on affinity tag compounds in the DNA modification identifier sequences (MoDIS) selected from the group consisting of biotin, biotin-TEG, wherein TEG is triethylene glycol, biotin-Cx, wherein Cx is an aliphatic linker, e.g. C₃₋₂₀, e.g. C₃, C₁₂ or C₁₈, biotin-BB, wherein BB is tertbutyl-benzoyl, for reducing undesired side reactions during oligonucleotide synthesis, or biotin-PC, wherein PC is a photocleavable linker). *(Olejniketal. 1995, (Nam et al. 2002, (Deng et al. 2003, (Fang et al. 2003, (Cozzolino et al. 2021)* Moreover, immuno-tags can be added to the DNA, e.g. digoxigenin, which can then be captured during a respective antibody in an affinity-based purification method. *(Kessler 1991)*

Optionally, the method of the present invention may further comprise the step of determining the position and/or the frequency of chemically modified triazole-linked DNA nucleotides comprising the fixed validation code (VC) and the unique randomized index code (RIC) for the localization, and/or quantification of chemical modifications in DNA sequences, optionally for allocating chemical modifications in DNA sequences to different cells and genomes.

In a further aspect the present invention relates to the use of the DNA modification identifier sequences (MoDIS) of the present invention, or the use of the method of the present invention for identifying chemical modifications, their position and/or frequency in the DNA of a biological sample, optionally a mammalian or a human biological sample.

The DNA modification identifier sequences (MoDIS) are valuable tools for practicing the present invention. In this regard they can form part of a kit of parts, the kit optionally comprising at least one of
(a) DNA modification identifier sequences (MoDIS) of the present invention and/or
(b) instructions for performing the method of the present invention, and optionally
(c) reagents suitable for use in the method of the present invention.

The MoDIS of the present invention have diagnostic utility. Therefore, a further aspect of the present invention is directed to a DNA modification identifier sequence (MoDIS) of the present invention for use in a diagnostic method for determining a disease or medical condition associated with chemically modified DNA, optionally selected from the group of diseases and medical conditions consisting of cancer, lung cancer, colon cancer, leukemia, glioma, esophageal cancer, hepatocellular carcinoma, skin cancer, neurological diseases, and aging-associated conditions. Additionally, the MoDIS of the present invention have utility in toxicity testing methods, optionally, for the assessment of safety of compounds.

The present invention also reads on a method for the identification of DNA damage and/or the diagnosis of a disease or medical condition in a mammalian, optionally human subject of interest comprising the steps
(A) obtaining a DNA sample from the subject of interest, optionally a sample comprising cells, blood, tissue, or saliva;
(B) detecting the number, sequence position and/or frequency of chemical modifications in the DNA of the sample by the method of the present invention;
(C) correlating the number, sequence position and/or frequency of chemical modifications in the DNA to a disease or medical condition of the subject of interest.

### Figures and exp. examples

In the following, the invention will be illustrated by way of representative figures and examples, none of which are to be interpreted as limiting the scope of the invention beyond the appended claims.

### Figures

**Fig. 1** is a schematic view of DNA damage modification identification in in the method employing MoDIS. The upper part shows chemical structures of 8-oxoG, AP-site and a phosphorylated single strand break (SSB). In the middle part, enzymes (FPG, ENDOIV, T4 PNK) convert these modifications to an SSB with a 3'-OH group, and an alkyne-modified nucleotide (prop-dNTP) is added to this position using a DNA polymerase as catalyst. In the lower part the alkyne is conjugated via copper click chemistry (CuAAC) with a MoDIS (VC-RIC-AS, black bar).

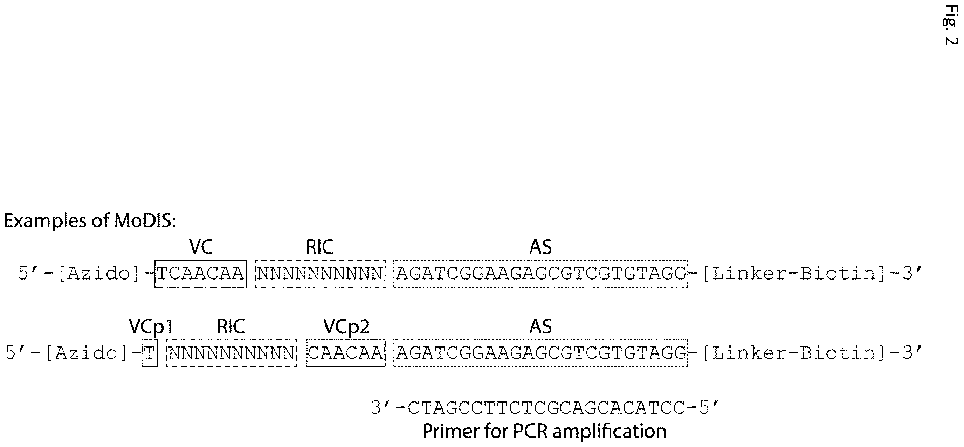

**Fig. 2** shows two representative and non-limiting examples of an MoDIS and a corresponding Primer for PCR amplification for use in the present invention (SeqIDNos.: 1, 2 and 3, numbered from top to bottom).

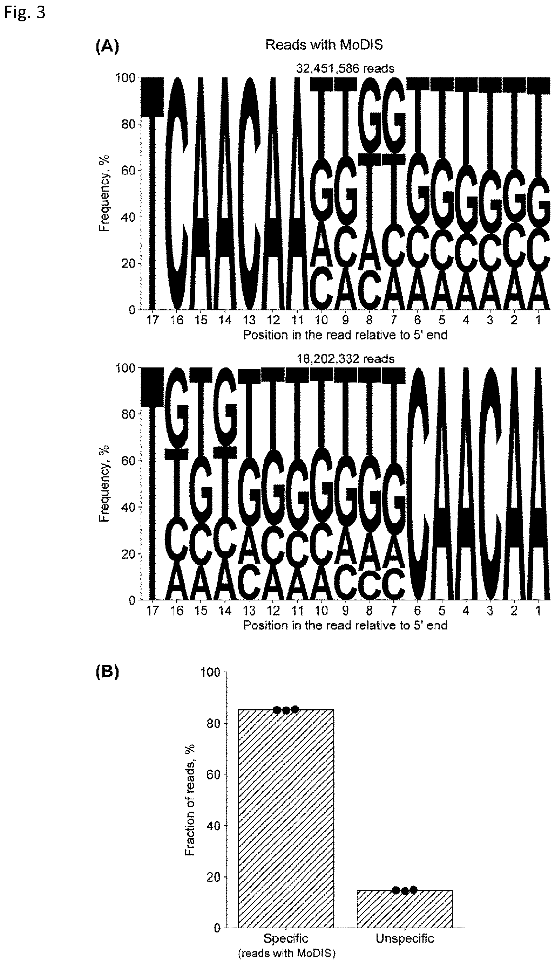

**Fig. 3****.** In the method employing MoDIS, sequencing reads that contain VC are validated as identifying a chemical modification (specific reads) and those readouts that do not contain VC are false positives (unspecific reads). (A) Sequence logos demonstrating the presence of VC and RIC in sequencing reads produced by the MoDIS-using method of genome-wide mapping of 8-oxoG. The frequency (Y axis) has the maximal value of 100 %, which means absolute conservation of a nucleotide at a certain position. The VC sequences in the used MoDIS (Fig. 2) are reflected in the fully conserved nucleotides of the reads. The 10 positions with variable nucleotide content reflect randomized sequence composition and correspond to RIC of the used MoDIS (Fig. 2). The sequence logos were build using the sequencing reads of one biological replicate. The reads used for the logos identify a chemical modification thanks to the presence of VC. (B) The fractions of the MoDIS-containing reads (with VC, see upper panel) and the unspecific reads lacking VC. Three biological replicates (circular markers) and their means ± s.d. (filled bars with error whiskers; error whiskers are narrow) of 8-oxoG mapping are shown. The reads in the upper panel correspond to one circular marker in the group of specific reads. Thanks to using MoDIS, the unspecific, i.e., artefactual reads, (around 15%) can be identified and removed.

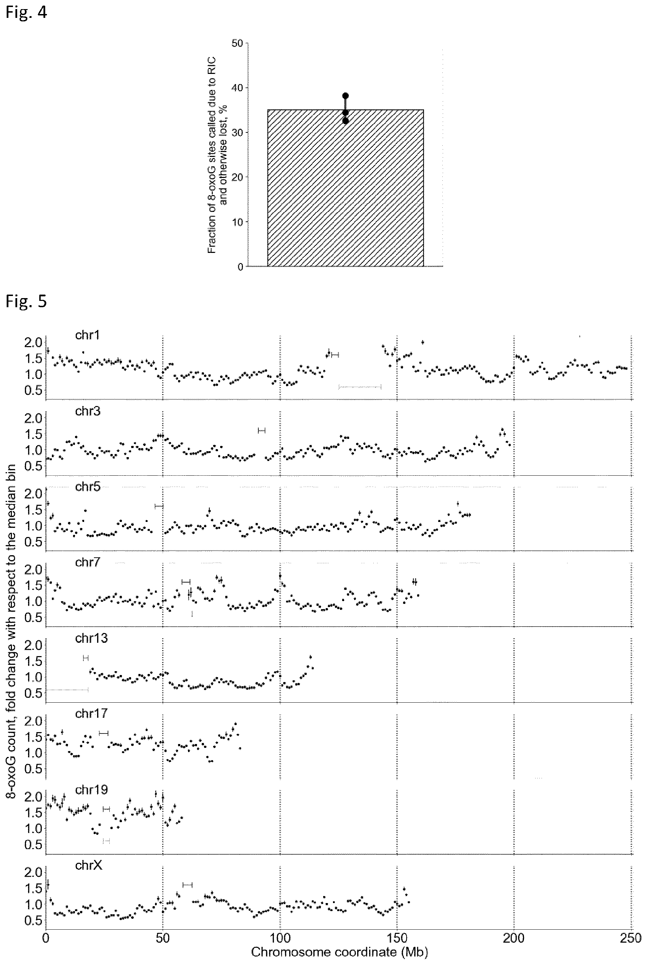

**Fig. 4****.** The presence of RIC in MoDIS increases by around 35% the number of identified 8-oxoG sites. The fraction relates the number of 8-oxoG-indetifying (*i.e*., VC-containing) deduplicated reads with RIC sequences and the number of the same reads with computationally removed RIC sequences. Three biological replicates (circular markers) and their means ± s.d. (filled bars with error whiskers) of 8-oxoG mapping are shown.
**Fig 5****.** Genome-wide mapping of 8-oxoG via the method employing MoDIS. The DNA modification pattern is shown for eight chromosomes. The 8-oxoG counts are calculated for bins of one million base pairs and normalized by dividing by genome-wide median value. Bin-related mean ± s.d. across three biological replicates are shown. Horizontal intervals with solid and dashed lines indicate respectively centromeres and reference-genome gaps (heterochromatin, short arms).

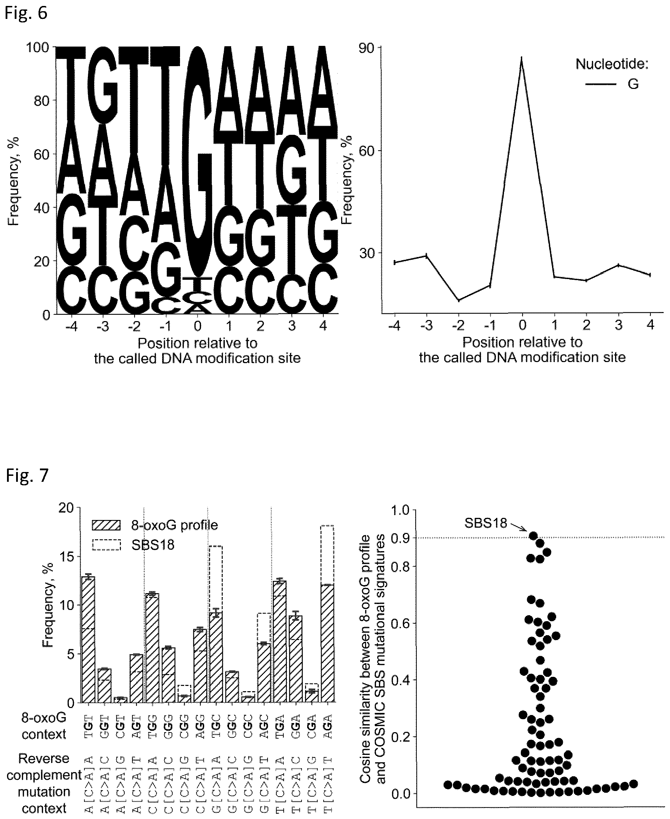

**Fig. 6****.** MoDIS and the method employing it identifies 8-oxoguanine (8-oxoG) with high specificity and at single nucleotide resolution. *Left panel:* sequence logo demonstrating high enrichment of guanine at called 8-oxoG sites and variable sequence context around the called sites. *Right panel:* guanine frequency is reproducibly high at the position of the called 8-oxoG. The error bars represent mean ± s. d. across biological replicates.
**Fig 7****.** Correlation of 8-oxoG distribution in the human cell line HAP-1 with the oxidative stress signature in human cancer. *Left panel:* The distribution of 8-oxoG in the local trinucleotide context. The filled bars and whiskers show the mean ± s.d. of the frequency of a specific trinucleotide in all trinucleotides with oxidized guanine in the second position (most of called damage sites are guanines, Fig. 6). The data of three biological replicates were used to calculate the means and s.d. The COSMIC mutational signature (v3.3) SBS18 (proposed etiology: damage by reactive oxygen species) is juxtaposed with the oxidative-damage profile by reverse-complementing their guanine-centered trinucleotides. The signature SBS18 is shown only for the C>A substitution subtype, which is putatively caused by 8-oxoG pairing with A and accounts for most substitutions in this signature. The presented frequencies of single base substitution C>A were normalized such that they add up to 100%. *Right panel:* The oxidative damage profile has the highest cosine similarity to SBS18 among all COSMIC single base substitution signatures. To juxtapose the oxidative damage profile with each SBS signature, we assumed that 8-oxoG causes C>A substitution on the opposite strand, reverse-complemented the trinucleotides with oxidized guanine in the second position and matched these reverse-complemented trinucleotides with the C>A substitution trinucleotides in the mutational signature. The trinucleotide frequencies of other substitution subtypes (C>G, C>T, T>A, T>C, T>G) in the mutational signature were matched with zero in the oxidative damage profile. The presented cosine similarity values are means across three biological replicates of oxidative DNA damage mapping.

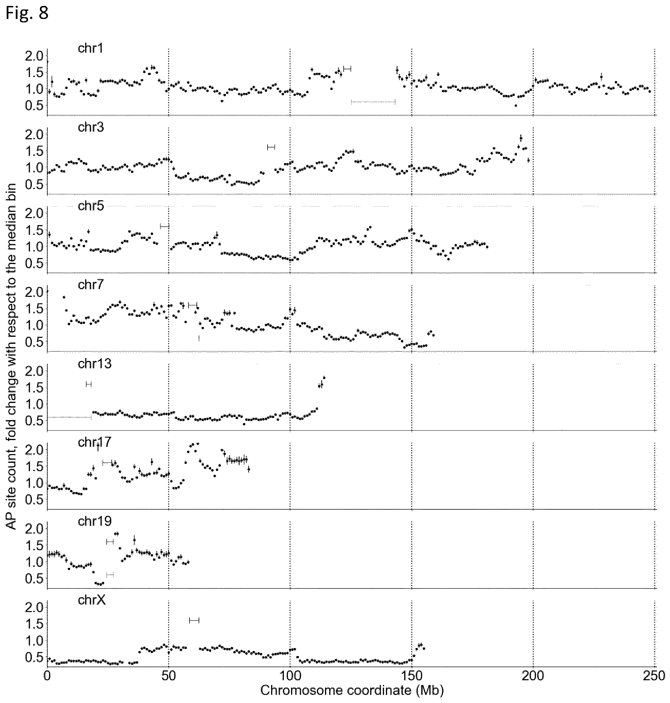

**Fig 8****.** Genome-wide mapping of abasic sites in cells exposed to irofulven via the method employing MoDIS. The DNA modification pattern is shown for eight chromosomes. The abasic-site counts are calculated for bins of one million base pairs and normalized by dividing by genome-wide median value. Bin-related mean ± s.d. across three biological replicates are shown. Horizontal intervals with solid and dashed lines indicate respectively centromeres and reference-genome gaps (heterochromatin, short arms).

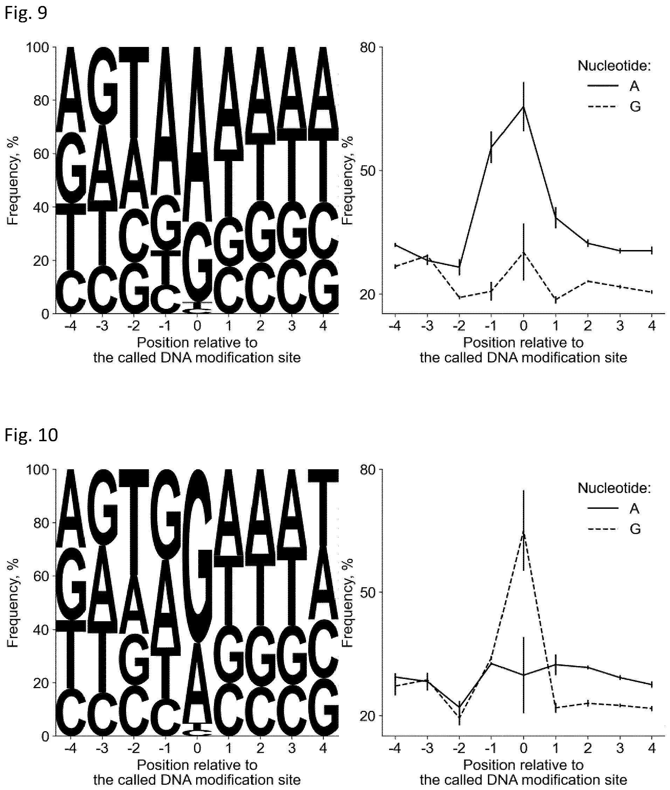

**Fig. 9****.** MoDIS and the method employing it identifies with high specificity and single-nucleotide resolution the abasic sites originating from depurination of adducts between DNA and irofulven (6-hydroxymethylacylfulvenein, HMAF) in U2OS human cell line. *Left panel:* sequence logo demonstrating high enrichment of adenine and smaller occurrence of guanine at called abasic sites and variable sequence context around the called sites. *Right panel:* adenine frequency is consistently higher than guanine frequency at the position of the called abasic sites. The error bars represent mean ± s.d.
**Fig. 10****.** MoDIS and the method employing it identifies that spontaneous depurination happens to guanine more frequently than to adenine in U2OS human cell line. *Left panel:* sequence logo demonstrating high enrichment of guanine at called abasic sites and variable sequence context around the called sites. *Right panel:* guanine frequency is reproducibly higher at the position of the called abasic sites. The error bars represent mean ± s. d.

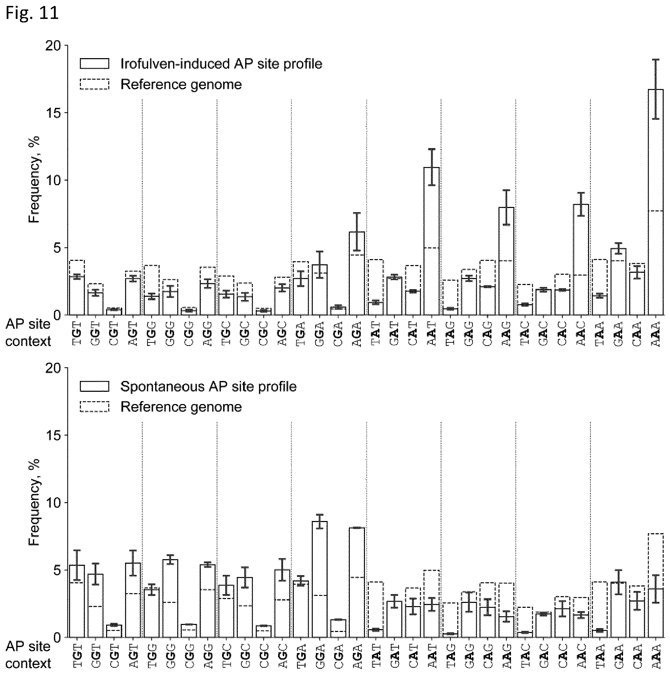

**Fig. 11****:** Irofulvene-induced abasic (AP) site distribution differs significantly from background abasic site profile in U2OS cells. The distribution of abasic sites in the local trinucleotide context. The solid-line bars and whiskers show the mean ± s.d. of the frequency of a specific trinucleotide in all trinucleotides with adenine or guanine in the second position (most of called damage sites, Fig. 9 and 10). The means and s.d. were calculated using the data of three biological replicates of irofulven exposure experiment (*upper panel*) and two biological replicates of the respective control experiment (*lower panel*)*.* The reference genome bars show the frequency of a specific trinucleotide in all trinucleotides with adenine or guanine in the second position as identified in the GRCh38 human reference genome, which was used to align sequencing reads.

### Examples

### Example 1 - 8-Oxoguanosine (8-oxoG) can be specifically sequenced in the genome of HAP1 cells Experimental procedure

*H₂O used in the experiment was purified by a Milli-Q^{®} IQ 7000 system (Merck, USA). All oligonucleotide DNA was synthesized by Eurogentec, Belgium. Oligonucleotides were received in a dried state, dissolved in Tris- (TE) buffer (10 mM Tris, pH 7.0-8.0, 1 mM ethylenediaminetetraacetic acid) and stored at -20°C.*

HAP1 cells (Horizon Discovery Ltd., United Kingdom) were cultivated in Iscove's Modified Dulbecco's Medium (Gibco/ThermoFisher Scientific, USA) supplemented with 10% fetal bovine serum (Gibco/ThermoFisher Scientific, USA) and 1% penicillin-streptomycin (Gibco/ThermoFisher Scientific, USA) at 37°C, 5% CO₂, 3% O₂. Cells were harvested by removing the medium, washing once with 10 mL Dulbecco's phosphate-buffered saline (Gibco/ThermoFisher Scientific, USA) followed by addition of 1 mL 0.25% Trypsin EDTA solution (Gibco/ThermoFisher Scientific, USA). After an incubation of 5 min at 37 °C, 9 mL medium was added, cells resuspended and transferred to a Falcon centrifugal tube. Cells were centrifuged (4°C, 0.3 rcf, 5 min) and the supernatant removed. The cell pellet was either stored at -20°C or directly used for DNA extraction.

*To minimize artefactual damage in the library preparation process, DNA samples were inverted or flicked instead of mixed with a pipette until the click reaction step.*

To block background modifications as abasic sites or strand breaks, 1 µL Endonuclease IV (ENDOIV) (10 U/µL, New England Biolabs, USA) was added to 5 µg freshly extracted genomic DNA (gDNA) from HAP1 pellets in 1x ThermoPol Buffer (New England Biolabs, USA) in a final volume of 20 µL. After 20 min incubation at 37°C, 2.5 µL of a dideoxynucleotide triphosphate mix (2.5 mM in H₂O, Jena Biosciences, Germany), 0.5 µL 10× ThermoPol Buffer (New England Biolabs, USA), 1.8 µL of H₂O and 0.2 µL Therminator IX (10 U/µL, New England Biolabs, USA) was added followed by a 10 min incubation at 60°C. 25 µL H₂O and 80 µL Pronex^{®} Magnetic Beads (Promega, USA) were added for purification following manufacturer's instructions with an elution volume of 22 µL. To label oxidative modifications, 1 µL formamidopyrimidine-DNA-glycosylase (FPG) (8 U/µL, New England Biolabs, USA), 1 µL ENDOIV (10 U/µL, New England Biolabs, USA) and 3 µL 10x NEBuffer 2 (New England Biolabs, USA) were added for final volume of 30 µL. The reaction mixture was incubated for 1h at 37°C, immediately followed by adding 3.5 µL 3'-(O-propargyl)-2'-deoxyguanosine triphosphate (2.5 mM in H₂O, Jena Biosciences, Germany), 3 µL 10x ThermoPol buffer (New England Biolabs, USA), 3 µL H₂O and 0.2 µL Therminator IX (10 U/µL, New England Biolabs, USA). After 10 min incubation at 60°C, gDNA was purified by adding 15 µL H₂O and using Pronex^{®} Magnetic Beads (Promega, USA) following manufacturer's instructions using an elution volume of 50 µL. Sonication of gDNA was performed using a Q800R2 sonicator (QSonica, USA) with the following settings: 4°C, 20% amplitude, pulse 15 s on, 5 s off, total 5 min. The resulting DNA fragments were analyzed on a Tapestation 2200 using a High Sensitivity D100 ScreenTape (Agilent Technologies, USA). DNA fragments were purified using Pronex^{®} Magnetic Beads (Promega, USA) purification following manufacturer's instructions with a sample:beads ratio of 1:2 and 50 µL elution volume. Samples were quantified using a Quantus^{™} Fluorometer (Promega, USA) with QuantiFluor^{®} ONE dsDNA Dye (Promega, USA). For adapter ligation, the oligonucleotides p5_01 and p7_01 were annealed by mixing them in annealing buffer consisting of 10 mM Tris (pH 7.5 - 8.0, Thermo Fisher Scientific, USA), 50 mM NaCl (Sigma Aldrich, USA) and 1 mM ethylenediaminetetraacetic acid (Thermo Fisher Scientific, USA). NEBNext^{®} Ultra^{™} II DNA Library Prep Kit for Illumina^{®} (New England Biolabs, USA) was utilized using 1 µg of gDNA and the pre-annealed adapter (15 µM). The manufacturer's instruction was followed without performing the USER enzyme step. The sample was purified using Pronex^{®} Magnetic Beads (Promega, USA) in sample:beads ratio 1:2 and elution volume of 50 µL. Successful adapter ligation was checked on a TapeStation 2200 with a High Sensitivity D100 ScreenTape (Agilent Technologies, USA). The sample volume was then condensed to 6 µL using a vacuum concentrator. To denature gDNA, the sample was heated to 95°C for 2 min and rapidly cooled on ice. Copper-catalyzed azide-alkyne cycloaddition (CuAAC) was performed using 2 µL MoDIS-CuAAC-Mix, which consists of a 100 µM equimolar mixture of oligonucleotides MoDIS_01 and MoDIS_02, 4 µL DMSO (Sigma Aldrich, USA), 2 µL premixed CuSO₄:THPTA (20 mM Cu²⁺ in H₂O and 200 mM THPTA in H₂O; THPTA from Lumiprobe, Germany; CuSO₄ from Sigma Aldrich, USA) and 2 µL sodium phosphate buffer (1 M, pH 7.0; Sigma Aldrich, USA). The reaction was started by adding 2 µL sodium ascorbate (400 mM in H₂O, freshly prepared; Sigma Aldrich, USA) and incubated for 60 min at 37°C. Purification was done using Pronex^{®} Magnetic Beads (Promega, USA) in a sample:beads ratio of 1:2 and 30 µL elution volume. Biotin enrichment of sample DNA was performed using Dynabeads^{™} MyOne^{™} Streptavidin C1 (ThermoFisher, USA). First, Bind&Wash buffer was prepared according to the manufacturer's protocol including 0.05 % Tween^{™} 20 (Sigma Aldrich, USA). Then, 15 µL Dynabeads^{™} were transferred to a fresh tube, washed three times with 200 µL 1x Bind&Wash buffer and resuspended in 30 µL 2x Bind&Wash buffer. The DNA sample was denatured by heating to 95°C for 2 min and rapid cooling on ice. Then, 30 µL sample was mixed with 30 µL of the prepared Dynabeads^{™} and rotated for 15 min at RT. Afterwards, the Dynabeads^{™} were washed 3x with 1x Bind&Wash buffer by resuspending the beads, 3 min rotation and 1 min pelleting on a magnetic rack. A final wash was performed with elution buffer (Promega, USA) and Dynabeads^{™} were resuspended in 20 µL elution buffer. To amplify the biotin-enriched material, a PCR was performed by adding 5 µL 10x ThermoPol buffer (New England Biolabs, USA), 5 µL deoxynucleotide triphosphate mix (2 mM in H₂O; New England Biolabs, USA), 1 µL MgSO₄ (100 mM in H₂O; Sigma Aldrich, USA), 1 µL ex_MoDIS_01 (10 µM), 1 µL ex_P7_01 (10 µM) and 0.5 µL Vent (exo-) DNA Polymerase (2 U/µL, New England Biolabs, USA). The PCR program comprised an initial denaturation step (95°C, 120 s), followed by 5 cycles of denaturation (95°C, 20 s), annealing (59°C, 20 s) and elongation (72°C, 60 s) and a final elongation step (72°C, 300 s). The sample was purified with Pronex^{®} Magnetic Beads (Promega, USA) in a sample:beads ratio 1:2 and an elution volume of 30 µL. To check for correct ligation of the sequencing adapters, a qPCR was performed. To a DNA mix (1 µL DNA sample, 5 µL H₂O) a qPCR mix consisting of 2.4 µL 5x Q5 Reaction Buffer (New England Biolabs, USA), 0.48 µL H₂O, 1.2 µL deoxynucleotide triphosphate mix (2 mM in H₂O; New England Biolabs, USA), 0.6 µL i5_lndex (10 µM) and 0.6 µL i7_lndex (10 µM), 0.6 µL 20x Evagreen^{®} (Biotium, USA) and 0.12 µL Q5 High Fidelity DNA Polymerase (2 U/µL; New England Biolabs, USA) was added. The qPCR program comprised an initial denaturation step (98°C, 30 s), 40 cycles of denaturation (98°C, 10 s), annealing (68°C, 30 s) and elongation (72°C, 30 s), and a final elongation step (72°C, 120 s). Green fluorescence was recorded.

Final library amplification and indexing was performed by adding 25 µL NEBNext^{®} Ultra^{™} II Q5^{®} Master Mix (New England Biolabs, USA), 2.5 µL i5_lndex (10 µM) and 2.5 µL i7_lndex (10 µM) to 20 µL sample. The PCR program comprised an initial denaturation step (98°C, 30 s), 10 cycles of denaturation (98°C, 10 s), annealing (68°C, 30 s) and elongation (72°C, 20 s), and a final elongation step (72°C, 120 s). The sample was purified with Pronex^{®} Magnetic Beads (Promega, USA) in a sample:beads ratio 1:2 and an elution volume of 20 µL. Samples were quantified using a Quantus^{™} Fluorometer (Promega, USA) with QuantiFluor^{®} ONE dsDNA Dye (Promega, USA). If the total sample amount was lower than 20 ng, additional PCR cycles were performed as described before to reach the desired sample amount. Finally, PCR samples were purified with the Pronex^{®} Magnetic Beads dual size selection protocol using a sample:beads ratio of 1:1, followed by a sample:beads ratio of 0.5:1 to exclude fragments longer than 1000 bp and shorter than 250 bp. The final library was quality-checked on a TapeStation 2200 with a High Sensitivity D100 ScreenTape (Agilent Technologies, USA). The final sample concentration was adjusted to a concentration of 4 ng/µL and sequenced on an NovaSeq 6000 Sequencing System (Illumina, USA) with 70 mio reads per sample.

**Oligonucleotide sequences in 5'-3'-notation**

| **Identifier** | **Sequence** |
|---|---|
| MoDIS_01 | N3-TNNNNNNNNNNCAACAAAGATCGGAAGAGCGTCGTGTAGG-TEG-Biotin, SEQ ID NO: 4 |
| MoDIS_02 | N3-TCAACAANNNNNNNNNNAGATCGGAAGAGCGTCGTGTAGG-TEG-Biotin, SEQ NO: 5 |
| Ex-MoDIS 01 | CCTACACGACGCTCTTCCGATC, SEQ ID NO: 3 |
| i5_01_Ind ex | |
| i7_01_Ind ex | AATGATACGGCGACCACCGAGATCTACACTATAGCCTACACTCTTTCCCTACACGACGCT CTTCCGATC*T, SEQ ID NO: 6 CAAGCAGAAGACGGCATACGAGATCGAGTAATGTGACTGGAGTTCAGACGTGTGCTCT TCCGATC*T, SEQ ID NO: 7 |
| p7_01 | AGACGTGTGCTCTTCCGATCTAGAAGGCCTAG*T, SEQ ID NO: 8 |
| p5_01 | Phos-CTAGGCCTTCTAAGGAGATGTTGATGTGCTGC, SEQ ID NO: 9 |
| Ex_P7_01 | AGACGTGTGCTCTTCCGATCTA, SEQ ID NO: 10 |

| | |
|---|---|
| (Phos = phosphorylation, N = random base, *= phosphothioester linkage, N3=azido, TEG = triethylene glycol linker) | |

### Data Evaluation/Bioinformatics

Sequencing read processing. After demultiplexing of sequencing data, each sample was represented by a fastq.gz file containing 101-nucleotide-long genomic reads. The quality of the data was checked using the program FastQC version 0.11.9 (below denoted in the manner: FastQC/0.11.9). Low-quality and adaptor-containing reads were removed via trimmomatic/0.38, using the following parameters: SE ILLUMINACLIP:Trimmomatic-0.39/adapters/TruSeq3-SE.fa:2:30:10 LEADING:3 TRAILING:3 SLIDINGWINDOW:4:15 MINLEN:101. The first 17 nucleotides corresponding to VC and RIC were clipped from the read sequences and appended to the read names via the tool *extract* of umi_tools/1.1.2 toolkit. The reads were mapped to human reference genome GRCh38 via bowtie2/2.3.5.1, using the pre-built bowtie2 index from https://genome-idx.s3.amazonaws.com/bt/GRCh38 noalt as.zip, trimming one nucleotide from reads' 5' end, which corresponds to a damage site, and applying otherwise default settings. Read duplicates were removed by the tool *dedup* of umi_tools/1.1.2 toolkit, grouping reads with the same R2 sequence stored in the read name (*method=unique*). samtools/1.12 was employed to remove unmapped reads, sort, index and generate statistics of bam files. bedtools2/2.29.2 was used to covert bam files to bed files. Each read represented one unit of DNA-modification signal, which we positioned at the nucleotide located immediately upstream of the 5' end of the read. Since a read was the reverse complement of the DNA fragment captured in the method employing MoDIS, the strand of the nucleotide bearing the signal was changed to the opposite to the one on which the respective read was mapped. Using a custom Python/3.7.4 script with the modules numpy/1.21.5 and pandas/0.25.1, we implemented the described DNA-modification-signal positioning. Software for mapped DNA-modification data analysis. The downstream analysis of DNA-modification data and their visualization were performed via custom Python/3.7.4 scripts and Jupyter notebooks employing the modules numpy/1.19.2, scipy/1.6.3, pandas/1.1.3, biopython/1.79, matplotlib/3.4.2 and seaborn/0.11.1 in Python/3.8.5 environment. Besides, bedtools2/2.29.2 was used to extract the sequence context of DNA breaks from the reference genome (GRCh38).

### Results

Genomic DNA (gDNA) was isolated from HAP1 cells. DNA breaks and AP sites, i.e., the most abundant forms of DNA damage confounding the oxidation mapping, were blocked by ddNTP insertion. Then, FPG was used to target oxidative damage sites and incorporated prop-dGTP. gDNA was sonicated and end-repaired, and adapter ligation was done using the NEBNext^{®} Ultra^{™} II DNA Library Prep Kit. The 3'-OH damage sites, originating from 8-oxoG excision and previously labeled with prop-dGTP, were ligated to two types of MoDIS (Fig. 2) by click chemistry. Since MoDIS are biotinylated, this allowed for a streptavidin-based enrichment following MoDIS ligation. Finally, DNA fragments were amplified and indexed using PCR, and sequenced. Raw sequencing data were processed by removing low-sequencing-quality reads and then mapped to the human reference genome. Quality checks performed during library preparation indicated a successful DNA-modification labeling procedure. For example, the Tapestation-based gel analysis showed an efficient sonication to a fragment size of around 350 bp and a change in size distribution after adapter ligation. qPCR amplification confirmed the presence of MoDIS and the quantity of MoDIS-ligated DNA samples; however, amplification could also be observed in non-adapter ligated DNA samples due to unspecific primer binding. Therefore, definite results are only visible after analyzing the sequencing data of the final DNA library produced in the method employing MoDIS (Fig. 1 and 2).

VC and RIC were identified in sequencing reads, which proved that the library preparation was successful and MoDIS were incorporated (Fig. 3, A). Sequencing reads that contained VC were validated as identifying a chemical modification (specific reads) and those reads that did not contain VC were false positives (unspecific reads). Around 15% of sequencing reads were thus identified as false positives (Fig. 3, B) and removed, which is an innovation compared to the state of the art and increases the accuracy of identifying, localizing, and quantifying 8-oxoG in DNA. Furthermore, it was found that the usage of RIC in MoDIS increased the number of identified 8-oxoG sites by around 35%, as compared to the case of not employing RIC (Fig. 4). The method using MoDIS allowed to identify, localize and quantify 8-oxoG in a genome-wide fashion throughout chromosomes (Fig. 5). The specificity for 8-oxoG and single-nucleotide resolution of the method employing MoDIS were confirmed by identifying mostly guanine at the position of the called DNA-modification site (Fig. 6).

To check if the method based on MoDIS could show validated DNA oxidation patterns, the dependency of 8-oxoG level on the local sequence context (Fig. 7, left panel) was analyzed and this damage profile was compared with single base substitution (SBS) signatures of the Catalogue of Somatic Mutations in Cancer (COSMIC). It was found that the distribution of 8-oxoG across trinucleotides has the highest level of similarity to the mutational signature SBS18 (Fig. 7), whose etiology is linked to reactive oxygen species (ROS)-induced damage and validated in experiments with human induced pluripotent stem cells exposed to potassium bromate generating hydroxyl radicals. *(Alexandrov et al. 2013, (Kucab et al. 2019)* Despite the overall high similarity between the damage profile and the signature SBS18 (Fig. 7, right panel), the mutation frequency is noticeably higher than the oxidation frequency at specific trinucleotides, such as TGC and AGA, which may reflect an impaired 8-oxoG repair in the respective sequence context in cells. On the contrary, TGT trinucleotide demonstrates a drop of the frequency of mutation relative to oxidation, potentially reflecting efficient repair in cells. Thus, the method based on MoDIS produced results that are consistent with existing knowledge of DNA oxidation patterns and provided additional insight into DNA damage formation and repair.

The DNA-modification data generated by the method based on MoDIS is highly reproducible across biological replicates (Fig. 3-7, small standard deviation).

### Example 2 - Abasic (apurinic, AP) sites can be specifically sequenced in the genome of U2OS cells

### Experimental procedure

*H₂O used in the experiment was purified by a Milli-Q^{®} IQ 7000 system (Merck, USA). All oligonucleotide DNA was synthesized by Eurogentec, Belgium. Oligonucleotides were received in a dried state, dissolved in Tris- (TE) buffer (10 mM Tris, pH 7.0-8.0, 1 mM ethylenediaminetetraacetic acid) and stored at -20°C.*

U2OS cells were cultivated in Dulbecco's Modified Eagle Medium High Glucose, GlutaMAX^{™} Supplement Pyruvate (Gibco/ThermoFisher Scientific, USA) supplemented with 10% fetal bovine serum (Gibco/ThermoFisher Scientific, USA) and 1% penicillin-streptomycin (Gibco/ThermoFisher Scientific, USA) at 37°C, 5% CO₂, 3% O₂. Treatment media was prepared by adding 3 µL 50 mM (±)-hydroxymethylacylfulvene (HMAF, irofulven)solution to 10 mL medium. Control media was prepared by adding 3 µL DMSO (Sigma Aldrich, USA) to 10 mL medium. Solutions were sterile-filtered, added to the cell dishes, and then incubated for 4 h at 37°C. Cells were harvested by removing the medium, washing once with 10 mL Dulbecco's phosphate-buffered saline (Gibco/ThermoFisher Scientific, USA) followed by addition of 1 mL 0.25% Trypsin EDTA solution (Gibco/ThermoFisher Scientific, USA). After an incubation of 5 min at 37°C, 9 mL medium was added, cells resuspended and transferred to a Falcon centrifugal tube. Cells were centrifuged (4°C, 0.3 rcf, 5 min) and the supernatant removed. The cell pellet was either stored at -20°C or directly used for DNA extraction.

*To minimize artefactual damage in the library preparation process, DNA samples were inverted or flicked instead of mixed with a pipette until the click reaction step.*

Apurinic (AP) sites were induced by adding H₂O to 5 µg freshly extracted genomic DNA (gDNA) to give a final volume of 20 µL and incubating at 37°C for 18 h.
To block background strand breaks, 1 µLT4 polynucleotide kinase (T4 PNK) (10 U/µL, New England Biolabs, USA) was added to the gDNA in 1x NEBuffer 2 (New England Biolabs, USA) in a final volume of 25 µL. After 20 min incubation at 37°C, 3 µL of a dideoxynucleotide triphosphate mix (2.5 mM in H₂O, Jena Biosciences, Germany), 0.5 µL 10x NEBuffer 2 (New England Biolabs, USA), 1.3 µL of H₂O and 0.2 µL Therminator IX (10 U/µL, New England Biolabs, USA) was added followed by a 10 min incubation at 60°C. 20 µL H₂O and 80 µL Pronex^{®} Magnetic Beads (Promega, USA) were added for purification following manufacturer's instructions with an elution volume of 22 µL. To label AP sites, 1 µL Endonuclease IV (ENDOIV) (10 U/µL, New England Biolabs, USA) and 3 µL 10x ThermoPol buffer (New England Biolabs, USA) were added for final volume of 30 µL. The reaction mixture was incubated for 1 h at 37°C, immediately followed by adding 4 µL 3'-(O-propargyl)-2'-deoxyguanosine triphosphate (2.5 mM in H₂O, Jena Biosciences, Germany), 4 µL 3'-(O-propargyl)-2'-deoxyadenosine triphosphate (2.5 mM in H₂O, Jena Biosciences, Germany), 1 µL 10× ThermoPol buffer (New England Biolabs, USA), 0.8 µL H₂O and 0.2 µL Therminator IX (10 U/µL, New England Biolabs, USA). After 10 min incubation at 60°C, gDNA was purified by adding 10 µL H₂O and using Pronex^{®} Magnetic Beads (Promega, USA) following manufacturer's instructions using an elution volume of 50 µL. Sonication of gDNA was performed using a Q800R2 sonicator (QSonica, USA) with the following settings: 4°C, 20% amplitude, pulse 15 s on, 5 s off, total 5 min. The resulting DNA fragments were analyzed on a Tapestation 2200 using a High Sensitivity D100 ScreenTape (Agilent Technologies, USA). DNA fragments were purified using Pronex^{®} Magnetic Beads (Promega, USA) purification following manufacturer's instructions with a sample:beads ratio of 1:2 and 50 µL elution volume. Samples were quantified using a Quantus^{™} Fluorometer (Promega, USA) with QuantiFluor^{®} ONE dsDNA Dye (Promega, USA). For adapter ligation, the oligonucleotides p5_01 and p7_01 were annealed by mixing them in annealing buffer consisting of 10 mM Tris (pH 7.5 - 8.0, Thermo Fisher Scientific, USA), 50 mM NaCl (Sigma Aldrich, USA) and 1 mM ethylenediaminetetraacetic acid (Thermo Fisher Scientific, USA). NEBNext^{®} Ultra^{™} II DNA Library Prep Kit for Illumina^{®} (New England Biolabs, USA) was utilized using 1 µg of gDNA and the pre-annealed adapter (15 µM). The manufacturer's instruction was followed without performing the USER enzyme step. The sample was purified using Pronex^{®} Magnetic Beads (Promega, USA) in sample:beads ratio 1:2 and elution volume of 50 µL. Successful adapter ligation was checked on a TapeStation 2200 with a High Sensitivity D100 ScreenTape (Agilent Technologies, USA). The sample volume was then condensed to 6 µL using a vacuum concentrator. To denature gDNA, the sample was heated to 95°C for 2 min and rapidly cooled on ice. Copper-catalyzed azide-alkyne cycloaddition (CuAAC) was performed using 2 µL MoDIS-CuAAC-Mix, which consists of a 100 µM equimolar mixture of oligonucleotides MoDIS_01 and MoDIS_02, 4 µL DMSO (Sigma Aldrich, USA), 2 µL premixed CuSO₄:THPTA (20 mM Cu²⁺ in H₂O and 200 mM THPTA in H₂O; THPTA from Lumiprobe, Germany; CuSO₄ from Sigma Aldrich, USA) and 2 µL sodium phosphate buffer (1 M, pH 7.0; Sigma Aldrich, USA). The reaction was started by adding 2 µL sodium ascorbate (400 mM in H₂O, freshly prepared; Sigma Aldrich, USA) and incubated for 60 min at 37°C. Purification was done using Pronex^{®} Magnetic Beads (Promega, USA) in a sample:beads ratio of 1:2 and 30 µL elution volume. Biotin enrichment of sample DNA was performed using Dynabeads^{™} MyOne^{™} Streptavidin C1 (ThermoFisher, USA). First, Bind&Wash buffer was prepared according to the manufacturer's protocol including 0.05 % Tween^{™} 20 (Sigma Aldrich, USA). Then, 15 µL Dynabeads^{™} were transferred to a fresh tube, washed three times with 200 µL 1x Bind&Wash buffer and resuspended in 30 µL 2x Bind&Wash buffer. The DNA sample was denatured by heating to 95°C for 2 min and rapid cooling on ice. Then, 30 µL sample was mixed with 30 µL of the prepared Dynabeads^{™} and rotated for 15 min at RT. Afterwards, the Dynabeads^{™} were washed 3x with 1x Bind&Wash buffer by resuspending the beads, 3 min rotation and 1 min pelleting on a magnetic rack. A final wash was performed with elution buffer (Promega, USA) and Dynabeads^{™} were resuspended in 20 µL elution buffer. To amplify the biotin-enriched material, a PCR was performed by adding 5 µL 10x ThermoPol buffer (New England Biolabs, USA), 5 µL deoxynucleotide triphosphate mix (2 mM in H₂O; New England Biolabs, USA), 1 µL MgSO₄ (100 mM in H₂O; Sigma Aldrich, USA), 1 µL ex_MoDIS_01 (10 µM), 1 µL ex_P7_01 (10 µM) and 0.5 µL Vent (exo-) DNA Polymerase (2 U/µL, New England Biolabs, USA). The PCR program comprised an initial denaturation step (95°C, 120 s), followed by 5 cycles of denaturation (95°C, 20 s), annealing (59°C, 20 s) and elongation (72°C, 60 s) and a final elongation step (72°C, 300 s). The sample was purified with Pronex^{®} Magnetic Beads (Promega, USA) in a sample:beads ratio 1:2 and an elution volume of 30 µL. To check for correct ligation of the sequencing adapters, a qPCR was performed. To a DNA mix (1 µL DNA sample, 5 µL H₂O) a qPCR mix consisting of 2.4 µL 5x Q5 Reaction Buffer (New England Biolabs, USA), 0.48 µL H₂O, 1.2 µL deoxynucleotide triphosphate mix (2 mM in H₂O; New England Biolabs, USA), 0.6 µL i5_lndex (10 µM) and 0.6 µL i7_lndex (10 µM), 0.6 µL 20x Evagreen^{®} (Biotium, USA) and 0.12 µL Q5 High Fidelity DNA Polymerase (2 U/µL; New England Biolabs, USA) was added. The qPCR program comprised an initial denaturation step (98°C, 30 s), 40 cycles of denaturation (98°C, 10 s), annealing (68°C, 30 s) and elongation (72°C, 30 s), and a final elongation step (72°C, 120 s). Green fluorescence was recorded.

Final library amplification and indexing was performed by adding 25 µL NEBNext^{®} Ultra^{™} II Q5^{®} Master Mix (New England Biolabs, USA), 2.5 µL i5_lndex (10 µM) and 2.5 µL i7_lndex (10 µM) to 20 µL sample. The PCR program comprised an initial denaturation step (98°C, 30 s), 10 cycles of denaturation (98°C, 10 s), annealing (68°C, 30 s) and elongation (72°C, 20 s), and a final elongation step (72°C, 120 s). The sample was purified with Pronex^{®} Magnetic Beads (Promega, USA) in a sample:beads ratio 1:2 and an elution volume of 20 µL. Samples were quantified using a Quantus^{™} Fluorometer (Promega, USA) with QuantiFluor^{®} ONE dsDNA Dye (Promega, USA). If the total sample amount was lower than 20 ng, additional PCR cycles were performed as described before to reach the desired sample amount. Finally, PCR samples were purified with the Pronex^{®} Magnetic Beads dual size selection protocol using a sample:beads ratio of 1:1, followed by a sample:beads ratio of 0.5:1 to exclude fragments longer than 1000 bp and shorter than 250 bp. The final library was quality-checked on a TapeStation 2200 with a High Sensitivity D100 ScreenTape (Agilent Technologies, USA). The final sample concentration was adjusted to a concentration of 4 ng/µL and sequenced on an NovaSeq 6000 Sequencing System (Illumina, USA) with 70 mio reads per sample.

**Oligonucleotide sequences in 5'-3'-notation**

| **Identifier** | **Sequence** |
|---|---|
| MoDIS_01 | N3-TNNNNNNNNNNCAACAAAGATCGGAAGAGCGTCGTGTAGG-TEG-Biotin, SEQ ID NO: 4 |
| MoDIS_02 | N3-TCAACAANNNNNNNNNNAGATCGGAAGAGCGTCGTGTAGG-TEG-Biotin, SEQ ID NO: 5 |
| Ex-MoDIS_01 | CCTACACGACGCTCTTCCGATC, SEQ ID NO: 3 |
| i5_01_Ind ex | AATGATACGGCGACCACCGAGATCTACACTATAGCCTACACTCTTTCCCTACACGACGCT CTTCCGATC*T, SEQ ID NO: 6 |
| i7_01_Ind ex | CAAGCAGAAGACGGCATACGAGATCGAGTAATGTGACTGGAGTTCAGACGTGTGCTCT TCCGATC*T, SEQ ID NO: 7 |
| p7_01 | AGACGTGTGCTCTTCCGATCTAGAAGGCCTAG*T, SEQ ID NO: 8 |
| p5_01 | Phos-CTAGGCCTTCTAAGGAGATGTTGATGTGCTGC, SEQ ID NO: 9 |
| Ex_P7_01 | AGACGTGTGCTCTTCCGATCTA, SEQ ID NO: 10 |

| | |
|---|---|
| (Phos = phosphorylation, N = random base, *= phosphothioester linkage, N3=azido, TEG = triethylene glycol linker) *Data Evaluation*/*Bioinformatics* | |

### Data Evaluation/Bioinformatics

Sequencing read processing. After demultiplexing of sequencing data, each sample was represented by a fastq.gz file containing 101-nucleotide-long genomic reads. The quality of the data was checked using FastQC/0.11.9. Low-quality and adaptor-containing reads were removed via trimmomatic/0.38, using the following parameters: SE ILLUMINACLIP:Trimmomatic-0.39/adapters/TruSeq3-SE.fa:2:30:10 LEADING:3 TRAILING:3 SLIDINGWINDOW:4:15 MINLEN:101. The first 17 nucleotides corresponding to VC and RIC were clipped from the read sequences and appended to the read names via the tool *extract* of umi_tools/1.1.2 toolkit. The reads were mapped to human reference genome GRCh38 via bowtie2/2.3.5.1, using the pre-built bowtie2 index from https://genome-idx.s3.amazonaws.com/bt/GRCh38 noalt as.zip, trimming one nucleotide from reads' 5' end, which corresponds to a damage site, and applying otherwise default settings. Read duplicates were removed by the tool *dedup* of umi_tools/1.1.2 toolkit, grouping reads with the same R2 sequence stored in the read name (*method*=*unique*). samtools/1.12 was employed to remove unmapped reads, sort, index and generate statistics of bam files. bedtools2/2.29.2 was used to covert bam files to bed files. Each read represented one unit of DNA-modification signal, which we positioned at the nucleotide located immediately upstream of the 5' end of the read. Since a read was the reverse complement of the DNA fragment captured in the method employing MoDIS, the strand of the nucleotide bearing the signal was changed to the opposite to the one on which the respective read was mapped. Using a custom Python/3.7.4 script with the modules numpy/1.21.5 and pandas/0.25.1, the described DNA-modification-signal positioning was implemented. Software for mapped DNA-modification data analysis. The downstream analysis of DNA-modification data and their visualization were performed via custom Python/3.7.4 scripts and Jupyter notebooks employing the modules numpy/1.19.2, scipy/1.6.3, pandas/1.1.3, biopython/1.79, matplotlib/3.4.2 and seaborn/0.11.1 in Python/3.8.5 environment. Besides, bedtools2/2.29.2 was used to extract the sequence context of DNA breaks from the reference genome (GRCh38).

### Results

Genomic DNA (gDNA) was isolated from U2OS cells exposed to irofulven (6-hydroxymethylacylfulvenein, HMAF) or respective control (DMSO). DNA breaks, i.e., the most abundant form of DNA damage confounding the abasic (apurinic, AP) site mapping, were blocked by ddNTP insertion. Irofulven-DNA adducts were converted to AP sites by incubating at 37°C for 18 h. Then, we used Endonuclease IV (ENDOIV) to target AP sites and incorporated prop-dGTP and prop-dATP. gDNA was sonicated and end-repaired, and adapter ligation was done using the NEBNext^{®} Ultra^{™} II DNA Library Prep Kit. The 3'-OH damage sites, originating from AP site removal and previously labeled with prop-dGTP, were ligated to two types of MoDIS (Fig. 2) by click chemistry. Since MoDIS are biotinylated, this allowed for a streptavidin-based enrichment following MoDIS ligation. Finally, DNA fragments were amplified and indexed using PCR, and sequenced. Raw sequencing data were processed by removing low-sequencing-quality reads and then mapped to the human reference genome (GRCh38). Quality checks performed during library preparation indicated a successful MoDIS labeling procedure. For example, the Tapestation-based gel analysis showed an efficient sonication to a fragment size of around 350 bp and a change in size distribution after MoDIS ligation. qPCR amplification confirmed the presence of the MoDIS and the quantity of MoDIS-ligated DNA samples.

The method using MoDIS allowed for identifying, localizing and quantifying irofulven-induced AP sites in a genome-wide fashion throughout chromosomes (Fig. 8), which provided a completely different genome-wide landscape compared to 8-oxoG mapping (Fig. 5). The specificity and single-nucleotide resolution of the method employing MoDIS were confirmed by identifying mostly adenine and guanine at the position of the called DNA-modification sites in both irofulven-exposed and control conditions (Fig. 9-10). Moreover, MoDIS and the method employing it detected that the abasic site formation originating from depurination of irofulven-DNA adducts happened more frequently to adenine (Fig. 9), as compared to spontaneous abasic site formation more common for guanine (Fig. 10). The observation of the irofulven-induced abasic sites primarily at adenines (Fig. 9) agrees with existing knowledge that irofulven forms adducts more efficiently with adenine than with guanine (*Tanasova et al. 2012*) and proves the specificity, single-nucleotide resolution and quantitative property of the MoDIS-utilizing method. Further, the dependency of AP site level on the local sequence context was analyzed and it was found that irofulvene-induced AP site distribution differs significantly from spontaneous (background) abasic site profile in U2OS cells (Fig. 11). There was evidence for sequence preference of irofulven modification of DNA, with the highest levels of modification in adenine dimers (Fig. 11, upper panel, trinucleotides AAT, AAG, AAC and AAA, where the second nucleotide is modified). Thus, the method based on MoDIS produced results that are consistent with existing knowledge of DNA modification by irofulven and provided additional insight into the mechanism of irofulven-induced damage formation.

### References

A. Al-Attar et al. Br. J. Cancer 2010 102(4): 704-709.
S. Alberti et al. Nucleic Acids Res. 1990 18(2): 351-353.
L. B. Alexandrov et al. Nature 2013 500(7463): 415-421.
J. M. Aliotta et al. Genet Anal 1996 12(5-6): 185-195.
I. Alseth et al. Mol Microbiol 2006 59(5): 1602-1609.
S. Berensmeier Appl. Microbiol. Biotechnol. 2006 73(3): 495-504.
N. E. Biolabs. (2023). "Q5® High-Fidelity DNA Polymerase." Retrieved 08.06.2023, 2023, from https://international.neb.com/products/m0491-q5-high-fidelity-dna-polymerase#Quality,%20Safety%20&%20Legal_Legal%20and%20Disclaimers.
L. Blanco et al. Proc Natl Acad Sci U S A 1984 81(17): 5325-5329.
M. Bollen et al. Crit. Rev. Biochem. Mol. Biol. 2000 35(6): 393-432.
R. J. Boorstein et al. J Biol Chem 2001 276(45): 41991-41997.
F. Boudsocq et al. Nucleic Acids Res 2001 29(22): 4607-4616.
K. Budelier et al. Curr Protoc Mol Biol 2001 Chapter 2: Unit2 1B.
V. Cameron et al. Biochem. 1977 16(23): 5120-5126.
B. Cao et al. Nucleic Acids Res. 2020 48(12): 6715-6725.
H. Cao et al. Not. Commun. 2019 10(1): 5799.
N. Chatterjee et al. Environ Mol Mutagen 2017 58(5): 235-263.
N. Chester et al. Anal Biochem 1993 209(2): 284-290.
C. J. Chetsanga et al. Biochem. 1981 20(18): 5201-5207.
A. Chien et al. J Bacteriol 1976 127(3): 1550-1557.
F. Cozzolino et al. J. Proteome Res. 2021 20(6): 3018-3030.
J. M. D'Alessio et al. Nucleic Acids Res 1988 16(5): 1999-2014.
M. a. S. Dairawan, P J Am. J. Biomed. Sci. Res. 2020 8(1): 39-45.
M. de Backer et al. J Mol Biol 2002 318(5): 1265-1274.
W.-G. Deng et al. Anal. Biochem. 2003 323(1): 12-18.
V. Derbyshire et al. Science 1988 240(4849): 199-201.
M. Dizdaroglu et al. Biochem. 1999 38(1): 243-246.
M. R. Ebrahimkhani et al. Proc Natl Acad Sci U S A 2014 111(45): E4878-4886.
A. H. EI-Sagheer et al. Chem Soc Rev 2010 39(4): 1388-1405.
A. H. EI-Sagheer et al. Q. Rev. Biophys. 2015 48(4): 429-436.
H. I. Elsner et al. DNA 1989 8(10): 697-701.
M. J. Engler et al. J Biol Chem 1983 258(18): 11165-11173.
S. Fang et al. Bioconjug. Chem. 2003 14(1): 80-85.
F. Faucher et al. J Mol Biol 2010 397(1): 46-56.
A. Free et al. J Biol Chem 2001 276(5): 3353-3360.
A. F. Gardner et al. Nucleic Acids Res 1999 27(12): 2545-2553.
A. F. Gardner et al. Front Mol Biosci 2019 6: 28.
S. J. Garger et al. Biochem Biophys Res Commun 1983 117(3): 835-842.
M. M. Haque et al. Sci. China Chem. 2014 57(2): 215-231.
Z. Hatahet et al. J Biol Chem 1994 269(29): 18814-18820.
W. A. M. Hoeijmakers et al. Nat. Protoc. 2011 6(7): 1026-1036.
H. W. Jannasch et al. Appl Environ Microbiol 1992 58(11): 3472-3481.
M. M. Kaplan J. Gastroenterol. 1972 62(3): 452-468.
C. Kessler Mol Cell Probes 1991 5(3): 161-205.
E. Kim et al. Chem Sci 2019 10(34): 7835-7851.
M. Kisiala et al. Nucleic Acids Res 2018 46(19): 10489-10503.
O. A. Kladova et al. J Mol Biol 2019 431(6): 1098-1112.
H. Kong et al. J Biol Chem 1993 268(3): 1965-1975.
J. E. Kucab et al. Cell 2019 177(4): 821-836.e816.
T. A. Kunkel et al. Methods Enzymol 1987 154: 367-382.
Y. Leitner-Dagan et al. J Natl Cancer Inst 2012 104(22): 1765-1769.
T. Lindahl et al. Cold Spring Harb Symp Quant Biol 2000 65: 127-133.
S. Ljungquist Methods Enzymol 1980 65(1): 212-216.
C. Mingard et al. Chem Soc Rev 2020 49(20): 7354-7377.
J.-M. Nam et al. JACS 2002 124(15): 3820-3821.
T. Notomi et al. Nucleic Acids Res 2000 28(12): E63.
S. Nwaka et al. J Biol Chem 1995 270(17): 10193-10198.
J. Olejnik et al. PNAS 1995 92(16): 7590-7594.
A. R. Poetsch et al. Genome Biol. 2018 19(1): 215.
M. A. Quail et al. Not. Methods 2012 9(1): 10-11.
M. T. Rahman et al. Anwer Khan Mod. Med. Coll. J. 2013 4(1): 30-36.
M. Rina et al. Eur J Biochem 2000 267(4): 1230-1238.
S. G. Rogers et al. Methods Enzymol 1980 65(1): 201-211.
J. Sambrook et al. CSH Protoc 2006 2006(1).
N. Sapojnikova et al. J. Biotechnol. 2017 256: 1-5.
A. H. Sarker et al. Prog Biophys Mol Biol 2021 164: 72-80.
A. A. Sartori et al. Nucleic Acids Res 2004 32(22): 6531-6539.
H. W. Servius et al. J Biol Chem 2023 299(1): 102756.
K. Shao et al. PLoS One 2011 6(9): e24910.
A. M. Sriramachandran et al. Mol Cell 2020 78(5): 975-985.e977.
J. A. Swenberg et al. Toxicol Sci 2011120 Suppl 1(Suppl 1): S130-145.
M. Tanasova et al. Chem. Rev. 2012 112(6): 3578-3610.
F. Tang et al. J Am Chem Soc 2022 144(1): 454-462.
A. Tubbs et al. Cell 2017 168(4): 644-656.
B. Uszczynska et al. Lob Chip 2012 12(6): 1151-1156.
P. A. van der Kemp et al. Proc Natl Acad Sci USA 1996 93(11): 5197-5202.
A. Winter et al. Synthesis 2009 2009(09): 1506-1512.
J. Wu et al. J Am Chem Soc 2018 140(31): 9783-9787.
Z. Yang et al. DNA Repair (Amst) 2017 52: 1-11.

## Claims

1. Modified DNA identifier sequences (MoDIS) having the structure
(a) 5'-VC - RIC - AS-3',
(b) 5'-RIC - VC - AS-3',
(c) 5'-VCp1 - RIC - VCp2 - AS-3',
(d) 5'-RICp1 - VC - RICp2 - AS-3', or
(e) 5'-VCpl - RICp1 - VCp2 - RICp2 - AS-3'
wherein
(i) VC is a validation code nucleotide sequence having 3 or more fixed nucleotides located upstream of RIC (a), downstream of RIC (b), or located in part upstream (VCp1) and located in part downstream of RIC (VCp2) (c);
(ii) RIC is a randomized index code nucleotide sequence having 3 or more randomly selected nucleotides (a-c), wherein the RIC can be located in part upstream (RICp1) and in part downstream (RICp2) of VC (d, e);
(iii) AS is an annealing site nucleotide sequence for specifically binding a primer for PCR amplification.
wherein
(a) the annealing site nucleotide sequence (AS) is attached to a marker and/or affinity tag compound for the identification, enrichment and/or purification of MoDIS-bound nucleotide sequences; and
(b) the nucleotide at the 5'-end of the MoDIS comprises a 5'-azido-modified moiety or a 5'-alkynyl-modified moiety.

2. The DNA modification identifier sequences (MoDIS) according to claim 1, wherein the randomized index code (RIC) is a nucleotide sequence having 5 to 200, 5 to 30, 6 to 15 or about 10 random DNA nucleotides.

3. The DNA modification identifier sequences (MoDIS) according to any one of claims 1 or 2, wherein the validation code (VC) is a nucleotide sequence having 4 to 400, 4 to 100, 4 to 20, 5 to 10 or about 7 random DNA nucleotides.

4. The DNA modification identifier sequences (MoDIS) according to any one of claims 1 to 3, wherein the annealing site (AS) sequence is a nucleotide sequence having 5 to 50, 10 to 50, 10 to 30, 18 to 30 or about 23 DNA nucleotides.

5. The DNA modification identifier sequences (MoDIS) according to any one of claims 1 to 4, wherein the annealing site nucleotide sequence AS specifically binds a primer of a polymerase for PCR amplification selected from group consisting of Vent (exo-) DNA polymerase, Deep Vent DNA Polymerase, Q5 high-fidelity DNA polymerase (New England Biolabs, USA), Therminator Polymerase, Therminator IX Polymerase, Klenow fragment, phi29 DNA Polymerase, Sulfolobus DNA Polymerase IV, Taq DNA Polymerase, BST DNA Polymerase (Full length or large fragment), Phusion DNA Polymerase, T7 DNA Polymerase, DNA Polymerase I, T4 DNA Polymerase.

6. The DNA modification identifier sequences (MoDIS) according to any one of claims 1 to 5, wherein the marker and/or affinity tag compound for the identification, enrichment and/or purification of a MoDIS-bound nucleotide sequence is selected from the group consisting of biotin, biotin-TEG, wherein TEG is triethylene glycol, biotin-C_{X}, wherein C_{X} is an aliphatic linker, optionally a C₃, C₁₂ or C₁₈ aliphatic linker, biotin-BB, wherein BB is tertbutyl-benzoyl; and biotin-PC, wherein PC is a photocleavable linker.

7. A method for detecting chemical modifications in DNA sequences comprising the steps
(a) Extraction of DNA from a biological sample;
(b) Enzymatic treatment of the extracted DNA with at least one protein for nucleotide excision, optionally selected from the group of proteins consisting of glycosylases, endonucleases, phosphatases, to remove chemically modified nucleotides and to yield an at least one-nucleotide-long gap in the double-stranded DNA with a free 3'-hydroxyl moiety;
(c) Introduction of 3'-(*O*- or S-alkynyl)-, optionally propargyl-modified nucleotides or 3'-azido-modified nucleotides at the free 3'-hydroxyl sites of the extracted DNA to provide 3'-(*O*- *or S*-alkynyl)-modified DNA, optionally propargyl-modified DNA, or 3'-azido-modified modified DNA, respectively;
(d) Optionally, ligation of adapters suitable for the sequencing technology used, optionally containing primer binding sites for loop-circle amplification for sequencing-by-synthesis approaches;
(d1) Reaction of the 3'-(*O*- or S-alkynyl)-, optionally propargyl-modified nucleotides of the extracted DNA with 5'-azido-modified nucleotides of the DNA modification identifier sequences (MoDIS) according to any one of claims 1 to 6 to form triazole-linked DNA nucleotide sequences comprising the DNA modification identifier sequences (MoDIS) linked via triazole to the extracted DNA fragments; or
(d2) Reaction of the 3'-azido-modified nucleotides of the extracted DNA with 5'-(*O*- or *S-*alkynyl)-, optionally 5'-propargyl-modified nucleotides of DNA modification identifier sequences (MoDIS) according to any one of claims 1 to 6 to form triazole-linked DNA nucleotide sequences comprising the DNA modification identifier sequences (MoDIS) linked via triazole to the extracted DNA fragments;
(e) Enrichment and/or purification of the triazole-linked DNA;
(f) Amplification of the triazole-linked DNA by polymerase chain reaction (PCR) by means of a primer binding to the Annealing site (AS) of the DNA modification identifier sequences (MoDIS); and
(g1) Optionally, indexing with additional commercially supplied adapters for the chosen sequencing technology
(g2) Sequencing of the amplified triazole-linked DNA nucleotides.

8. The method for detecting chemical modifications according to claim 7, wherein the biological sample comprises cells, tissue, blood, or saliva.

9. The method for detecting chemical modifications according to any of claims 7 or 8, wherein the extracted DNA is genomic DNA or mitochondrial DNA.

10. The method for detecting chemical modifications according to any of claims 7 to 9, wherein the at least one nucleotide excision protein is selected from the group consisting formamidopyrimidine DNA glycosylase (Fpg), human apurinic/apyrimidinic endonuclease (APE1), 8-oxo-guanine glycosylase 1 (OGG1), 8-oxo-guanine glycosylase 2 (OGG2), Endonuclease three homologue 1 (NTH1), Endonuclease VII-like 1 / Nei-like DNA glycosylase 1 (NEIL1), Endonuclease VII-like 2 / Nei-like DNA glycosylase 2 (NEIL2), Endonuclease VII-like 3 / Nei-like DNA glycosylase 3 (NEIL3), archaeal GO glycosylase (AGOG), Alkyladenine glycosylase / Methylpurine glycosylase (AAG/MPG), 3-meA glycosylase 1 (Tagl), DNA-3-methyladenine glycosylase 2 (AlkA), AlkC, AlkD, Uracil DNA N-glycosylase (UNG), Thymine DNA glycosylase (TDG), ss-selective monofunctional uracil DNA glycosylase 1 (SMUG1), and methyl-CpG-binding domain 4 (MBD4) and T4 polynucleotide kinase (T4 PNK), antarctic phosphatase, alkaline phosphatase, shrimp alkaline phosphatase and pyrophosphatase, as well as mutants of these.

11. The method for detecting chemical modifications according to any one of claims 7 to 10,
wherein
(i) the 3'-(*O*-alkynyl, optionally propargyl)-modified nucleotides of the extracted DNA are reacted with 5'-azido-modified nucleotides of the DNA modification identifier sequences (MoDIS) according to any one of claims 1 to 6, or
(ii) the 3'-azido-modified nucleotides of the extracted DNA are reacted with the 5'-(*O-*alkynyl, optionally propargyl)-modified nucleotides of the DNA modification identifier sequences (MoDIS) according to any one of claims 1 to 6,
to form triazole-linked DNA nucleotide sequences.

12. The method for detecting chemical modifications according to any one of claims 7 to 11, wherein enrichment and/or purification of the triazole-linked DNA nucleotides is based on marker and/or affinity tag compounds in the DNA modification identifier sequences (MoDIS) selected from the group consisting of biotin, biotin-TEG, wherein TEG is triethylene glycol, biotin-Cx, wherein Cx is an aliphatic linker, optionally a C₃, C₁₂ or C₁₈ aliphatic linker, biotin-BB, wherein BB is tertbutyl-benzoyl, and or biotin-PC, wherein PC is a photocleavable linker.

13. The method for detecting chemical modifications according to any one of claims 7 to 11, further comprising the step of determining the position and/or the frequency of chemically modified triazole-linked DNA nucleotides comprising the fixed validation code (VC) and the unique randomized index code (RIC) for the localization, and/or quantification of chemical modifications in DNA sequences, optionally for allocating chemical modifications in DNA sequences to different cells and genomes.

14. A use of the DNA modification identifier sequences (MoDIS) of any one of claims 1 to 6, or a method of any one of claims 7 to 13 for identifying chemical modifications, their position and/or frequency in the DNA of a biological sample, optionally a mammalian or a human biological sample.

15. A kit of parts comprising at least one of
(a) DNA modification identifier sequences (MoDIS) of any one of claims 1 to 6, and/or
(b) instructions for performing the method of any one of claims 7 to 13, and optionally
(c) reagents suitable for use in the method of any one of claims 7 to 13.

16. A DNA modification identifier sequence (MoDIS) of any one of claims 1 to 6 for use in (a) a diagnostic method for determining a disease or medical condition associated with chemically modified DNA, optionally selected from the group of diseases and medical conditions consisting of cancer, lung cancer, colon cancer, leukemia, glioma, esophageal cancer, hepatocellular carcinoma, skin cancer, neurological diseases, and aging-associated conditions; (b) in a toxicity testing method.

17. A method for the identification of DNA damage and/or the diagnosis of a disease or medical condition in a mammalian, optionally human subject of interest comprising the steps
(A) obtaining a DNA sample from the subject of interest, optionally a sample comprising cells, blood, tissue, feces or saliva;
(B) detecting the number, sequence position and/or frequency of chemical modifications in the DNA of the sample by the method of any one of claims 7 to 13;
(C) correlating the number, sequence position and/or frequency of chemical modifications in the DNA to a disease or medical condition of the subject of interest.
